(19) Europäisches Patentamt
European Patent Office
Office européen des brevets

(11) Veröffentlichungsnummer: **0 103 158**
**B1**

(12) # EUROPÄISCHE PATENTSCHRIFT

(45) Veröffentlichungstag der Patentschrift: **26.02.86**

(21) Anmeldenummer: **83107831.6**

(22) Anmeldetag: **09.08.83**

(51) Int. Cl.$^4$: **C 07 D 513/14,** A 61 K 31/435, A 61 K 31/55, C 07 D 495/04 // (C07D513/14, 333:00, 277:00, 233:00),(C07D513/14, 333:00, 277:00, 221:00),(C07D495/04, 333:00, 223:00)

(54) **Neue Thieno-thiazol-Derivate, Verfahren zu ihrer Herstellung und diese Verbindungen enthaltende Arzneimittel.**

(30) Priorität: **18.08.82 DE 3230696**

(43) Veröffentlichungstag der Anmeldung: **21.03.84 Patentblatt 84/12**

(45) Bekanntmachung des Hinweises auf die Patenterteilung: **26.02.86 Patentblatt 86/9**

(84) Benannte Vertragsstaaten: **AT BE CH DE FR IT LI LU NL SE**

(56) Entgegenhaltungen: **EP - A - 0 058 341**

(73) Patentinhaber: **Dr. Karl Thomae GmbH, Postfach 1755, D-7950 Biberach (Riss) (DE)**

(72) Erfinder: **Sauter, Robert, Dr. Dipl.-Chem., Albert-Schweitzer-Weg 9, D-7958 Laupheim (DE)**
Erfinder: **Griss, Gerhart, Dr. Dipl.-Chem., Schopperweg 1, D-7950 Biberach 1 (DE)**
Erfinder: **Grell, Wolfgang, Dr. Dipl.-Chem., Amriswilstrasse 7, D-7950 Biberach (DE)**
Erfinder: **Hurnaus, Rudolf, Dr. Dipl.-Chem., Silcherstrasse 19, D-7950 Biberach 1 (DE)**
Erfinder: **Kobinger, Walter, Prof. Dr., Belghofergasse 27, A-1121 Wien (AT)**
Erfinder: **Pichler, Ludwig, Dr., Gusshausstrasse 24/11, A-1040 Wien (AT)**

**EP 0 103 158 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Gegenstand der vorliegenden Erfindung sind neue Thieno-thiazol-Derivate der allgemeinen Formel

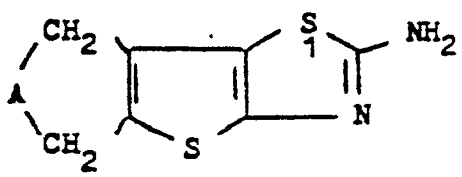

,(I)

deren Säureadditionssalze, insbesondere deren physiologisch verträgliche Säureadditionssalze mit anorganischen oder organischen Säuren, Verfahren zu ihrer Herstellung und diese Verbindungen enthaltende Arzneimittel.

Die neuen Verbindungen weisen wertvolle pharmakologische Eigenschaften auf, nämlich eine Wirkung auf das Herz und den Kreislauf, insbesondere jedoch eine herzfrequenzsenkende Wirkung.

In der obigen allgemeinen Formel bedeutet

A eine Gruppe der Formel

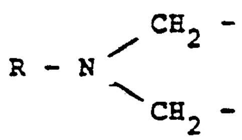

,

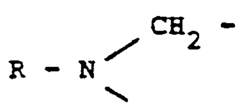

oder

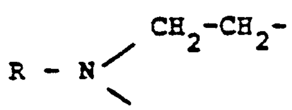

, wobei

R ein Wasserstoffatom, eine Alkyl- oder Alkanoylgruppe mit 1 bis 5 Kohlenstoffatomen, die Allyl-, Crotyl-, Penten- 2 bzw 3-ylgruppe, eine Alkoxycarbonylgruppe mit insgesamt 2 bis 5 Kohlenstoffatomen oder eine gegebenenfalls durch ein Halogenatom substituierte Aralkylgruppe mit 7 bis 11 Kohlenstoffatomen darstellt.

Gegenstand der vorliegenden Erfindung sind somit 2-Amino 6,7,8,9-tetrahydro-5H-thiazo1o [4',5':5,4] thieno [2,3-d] azepin-2-Amino-5,6,7,8-tetrahydro-thiazolo [4'5':5,4]thieno [3,2-c]-pyridin-, 2-Amino-5,6,7,8-tetrahydro-thiazolo [4',5':5,4]-thieno [2,3-c] pyridin-, 2-Amino-6,7,8,9-tetrahydro-5H-thia-zolo [4',5':5,4] thieno [2,3-c] azepin- und 2-Amino-6,7,8,9-tetrahydro-5H-thiazolo [4',5':5,4] thieno [3,2-c] azepin-Derivate der obigen allgemeinen Formel.

Für die bei der Definition des Restes R eingangs erwähnten Bedeutungen kommt beispielsweise die des Wasserstoffatoms, der Methyl-, Äthyl-, n-Propyl-, Isopropyl-, n-Butyl-, Isobutyl-, tert.-Butyl-, n-Pentyl-, Neopentyl-, tert.Pentyl-, Formyl-, Acetyl-, propionyl-, n-Butanoyl-, n-Pentanoyl-, 2-Methyl-propionyl-, Pivaloyl-, Allyl-, Crotyl-, Penten-2-yl-,

Penten-3-yl-, Methoxycarbonyl-, Äthoxycarbonyl-, n-Propoxycarbonyl-, Isopropoxycarbonyl-, n-Butoxycarbonyl-, Isobutoxycarbonyl-, tert.Butoxycarbonyl-, Benzyl-, 1-phenyläthyl-, 2-Phenyläthyl-, 3-Phenylpropyl-, Naphthylmethyl-, Chlorbenzyl-, Brombenzyl-, Fluorbenzyl-, 1-Chlorphenyläthyl-, 2-Bromphenyläthyl- oder 3-Chlorphenyl-propylgruppe in Betracht.

Bevorzugte Verbindungen sind jedoch die 2-Amino-6,7,8,9-tetrahydro-5H-thiazolo [4',5':5,4] thieno [2,3-d] azepin-, 2-Amino-5,6,7,8-tetrahydro-thiazolo [4',5':5,4] thieno [3,2-c]-pyridin-, 2-Azino-5,6,7,8-tetrahydro-thiazolo [4'5:5,4] -thieno [2,3-c] pyridin- und 2-Amino-6,7,8,9-tetrahydro-5H-thia-zolo [4',5':5,4] thieno [2,3-c] azepin-Derivate der obigen allgemeinen Formel, in der

R ein Wasserstoffatom, eine Alkylgruppe mit 1 bis 4 Kohlenstoffatomen, eine Alkanoylgruppe mit 1 bis 3

Kohlenstoffatomen, eine Alkoxycarbonylgruppe mit insgesamt 2 bis 4 Kohlenstoffatomen, eine gegebenenfalls durch ein Chloroder Bromatom substituierte Benzylgruppe oder eine Allylgruppe darstellt, und deren Säureadditionssalze, insbesondere deren physiologisch verträgliche Säureadditionssalze mit anorganischen oder organischen Säuren.

Besonders bevorzugte Verbindungen der vorliegenden Erfindung sind jedoch die 2-Amino-6,7,8,9-tetrahydro-5H-thiazolo-[4',5':5'4] thieno [2,3-d] azepin- und 2-Amino-5,6,7,8-tetrahydro-thiazolo [4',5':5,4] thieno [2,3-c] pyridin-Derivate der obigen allgemeinen Formel I, in der

R ein Wasserstoffatom, eine Alkylgruppe mit 1 bis 3 Kohlenstoffatomen, eine Allyl- oder Benzylgruppe darstellt, und deren physiologisch verträgliche Säureadditionssalze mit anorganischen oder organischen Säuren.

Erfindungsgemäß erhält man die neuen Verbindungen nach folgenden Verfahren:

a) Umsetzung eines gegebenenfalls im Reaktionsgemisch gebildeten Thiophens der allgemeinen Formel

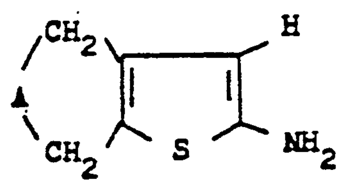

,(II)

in der

A wie eingangs definiert ist, mit gegebenenfalls im Reaktionsgemisch gebildetem Dirhodan.

Die Umsetzung wird vorzugsweise in einem Lösungsmittel oder Lösungsmittelgemisch wie Eisessig, Essigsäure/Wasser, Dioxan/ Essigsäure, Methanol, Tetrahydrofuran oder verdünnter Salzsäure bei Temperaturen zwischen -5 und 50°C, vorzugsweise jedoch bei Temperaturen zwischen 0 und 25°C, durchgeführt.

Hierbei wird das für die Umsetzung erforderliche Dirhodan zweckmäßigerweise durch Oxidation von Rhodanwasserstoff, beispielsweise mit Chlor, Brom, einem Schwermetallsalz wie Kupfersulfat oder durch anodische Oxidation, welcher seinerseits aus einem Salz des Rhodanwasserstoffs, vorzugsweise einem Alkalisalz wie dem Natrium- oder Kaliumsalz, mit Hilfe einer Säure wie Essigsäure, Salzsäure oder Schwefelsäure freigesetzt wird, oder durch Zersetzung eines entsprechenden Schwermetallsalzes wie Kupferdirhodanid hergestellt.

Desweiteren kann eine Verbindung der allgemeinen Formel II im Reaktionsgemisch durch Decarboxylierung einer entsprechenden 2-Amino-3-carboxy-Verbindung bzw. durch Hydrolyse und an-schließende Decarboxylierung einer entsprechenden Alkowycarbonylamino-Verbindung in Gegenwart einer Säure wie Triflouressigsäure, Eisessig/Bromwasserstoff oder Dioxan/Chlorwasser stoff hergestellt werden.

b) Zur Herstellung von Verbindungen der allgemeinen Formel der R eine gegebenenfalls durch eine Alkylgruppe mit 1 bis 4 Kohlenstoffatomen substituierte Methylgruppe darstellt:

Reduktion einer Verbindung der allgemeinen Formel

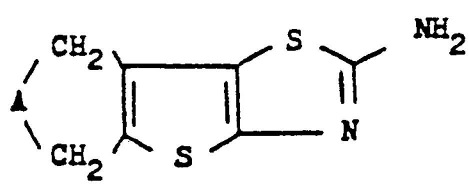

,(III)

in der

A eine Gruppe der Formel

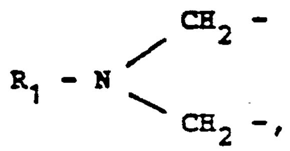 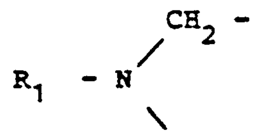 oder 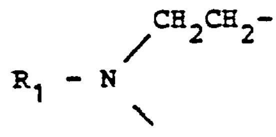

darstellt, wobei

$R_1$ eine Alkanoylgruppe mit 1 bis 5 Kohlenstoffatomen oder einen Kohlensäureesterrest wie die Äthoxycarbonyl-, Phenoxycarbonyl- oder Benzyloxycarbonylgruppe bedeutet.

Die Reduktion wird in Gegenwart eines Hydrids und zweckmäßiger-weise in einem geeigneten Lösungsmittel wie Diäthyläther, Tetrahydrofuran, Dioxan oder Pyridin/Tetrahydrofuran bei Temperaturen zwischen 0 und 100°C, vorzugsweise jedoch bei der Siedetemperatur des verwendeten Lösungsmittels, durchgeführt. Bevorzugt wird die Reduktion jedoch mit Lithiumaluminiumhydrid, Natriumaluminiumhydrid, Lithiumborhydrid, einem Natriumacyloxyborhydrid wie Natrium-acetoxyborhydrid oder Pyridin-Boran durchgeführt.

Die erhaltenen Verbindungen der allgemeinen Formel I lassen sich ferner in ihre Säureadditionssalze, insbesondere in ihre physiologisch verträglichen Salze mit anorganischen oder organischen Säuren überführen. Als Säuren kommen hierbei beispielsweise Salzsäure, Bromwasserstoffsäure, Schwefelsäure, Phosphorsäure, Milchsäure, Zitronensäure, Weinsäure, Maleinsäure oder Fumarsäure in Betracht.

Die als Ausgangsstoffe verwendeten Verbindungen der allgemeinen Formeln II und III, welche als Rohprodukte eingesetzt werden können, erhält man nach literaturbekannten Verfahren, z.B. durch Vilsmeier-Reaktion eines entsprechenden cyclischen Ketons, Überführung der so erhaltenen Chlor-formylverbindung über ihr Oxim in das entsprechende Nitril, Umsetzung des erhaltenen Nitrils mit einem Thioglykolester und anschließende Cyclisierung. Die so erhaltenen Ester können nach Hydrolyse anschließend decarboxyliert werden. Die Verbindungen der allgemeinen Formeln II und III sind teilweise Gegenstand der europäischen Anmeldung 82 100 745.7 vom 18.02.1981, welche am 25. 08. 1982 unter der Veröffentlichungsnummer EP-A- 0 058 341 veröffentlicht wird, bzw. können nach den dort beschriebenen Verfahren hergestellt werden. Die in dieser Publikation beschriebenen Endprodukte stellen bestimmte, in spezifisher Weise substituierte Thieno-azepine mit einer Wirkung auf den Intermediärstoffwechsel und gegen Thrombosen dar.

Dagegen weisen, wie bereits eingangs erwähnt, die neuen Verbindungen der allgemeinen Formel I und deren physiologisch verträgliche Säureadditionssalze wertvolle pharmakologische Eigenschaften auf, insbesondere neben einer Beeinflussung des Blutdrucks eine starke herzfrequenzsenkende Wirkung.

Beispielsweise wurden die Verbindungen

A = 7-Äthyl-2-amino-6,7,8,9-tetrahydro-5H-thiazolo [4',5':5,4]-thieno [2,3-d] azepin-dihydrochlorid,
B = 2-Amino-7-benzyl-6,7,8,9-tetrahydro-5H-thiazolo [4',5':5,4]-thieno [2,3-d] azepin,
C = 7-Allyl-2-amino-6,7,8,9-tetrahydro-5H-thiazolo [4',5':5,4]-thieno [2,3-d] azepin-dihydrochlorid,
D = 2-Amino-6,7,8,9-tetrahydro-7-isopropyl-5H-thiazolo-[4',5':5,4] thieno [2,3-d] azepin-dihydrochlorid,
E = 6-Äthyl-2-amino-5,6,7,8-tetrahydro-thiazolo [4',5':5,4]-thieno [2,3-c] pyridin-dihydrochlorid,
F = 2-Amino-5,6,7,8-tetrahydro-6-propyl-thiazolo [4',5:5,4]-thieno [2,3-c] apyridin-dihydrochlorid,
G = 2-Amino-5,6,7,8-tetrahydro-6-methyl-thiazolo [4',5':5,4]-thieno [2,3-c] pyridin-dihydrochlorid
H = 2-Amino-5,6,7,8-tetrahydro-5H-thiazolo [4',5':5,4] thieno- [2,3-d] azepin-dihydrochlorid

auf ihre biologischen Eigenschaften wie folgt untersucht:

**Wirkung auf Blutdruck und Herzfreguenz**

**Methodik:**

An der intakten, narkotisierten Ratte werden die Kreislaufveränderungen nach intravenöser Injektion der Testsubstanzen registriert.

**Versuchsbeschreibung:**

Männliche Ratten im Gewicht um 200 g werden mit Pentobarbital (50 mg/kg i.p.) narkotisiert. Die Tiere atmen spontan über eine eingebundene Trachealkanüle. Die Blutdruckmessung erfolgt aus einer A. carotis mit Hilfe

eines elektro-mechanischen Druckwandlers; die Pulswelle triggert einen Tachographen, der kontinuierlich die Herzfrequenz registriert. Beide Parameter werden auf einem Polygraphen aufgezeichnet. Testsubstanzen werden über einen Katheter in eine V. jugularis injiziert.

Die zu untezsuchenden Substanzen führten mit 3 mg/kg i.v. nach kurzdauernder initialer Blutdrucksteigerung zu längerdauernder Drucksenkung. Die Herzfrequenz war ausgeprägt und langdauernd gesenkt. Die maximalen Werte sind aus der nachstehenden Tabelle zu entnehmen:

| Substanz | Dosis mg/kg i.v. | Blutdruck in mm Hg pressorische Phase | depressorische Phase | Herzfrequenzsenkung Schläge/Minute |
|---|---|---|---|---|
| A | 3 | + 52 | - 18 | - 90 |
| B | 3 | | - 25 | - 95 |
| C | 3 | + 17 | - 15 | - 150 |
| D | 3 | + 23 | - 20 | - 78 |
| E | 3 | + 30 | - 29 | - 108 |
| F | 3 | + 16 | - 12 | - 65 |
| G | 3 | + 46 | - 30 | - 95 |
| H | 1,5 | + 38 | - 34 | - 100 |

**2. Wirkung an praesynaptischen α-Adrenoceptoren: Hemmung der elektrisch ausgelösten Tachycardie**

**Methodik:**

Am adrenergen Nervenende existiert ein Autoregulationssystem der Noradrenalinfreisetzung. Erregung praesynaptischer α -Adrenorezeptoren wirkt hemmend auf die Noradrenalinfreisetzung und damit einschränkend auf die adrenerge Neurotransmission. Dementsprechend wird bei Stimulierung dieser "feedback Rezeptoren" der Effekt einer gegebenen adrenergen Reizung am Erfolgsorgan abgeschwächt werden.

Am Modell der elektrisch gereizten Spinalratte wird die am Herzen elektrisch induzierte Tachykardie durch praesynaptisch α -mimetisch wirksame Substanzen abgeschwächt.

**Versuchsbeschreibung:**

Männliche Ratten im Gewicht von 350 - 400 g werden mit Pentobarbital (50 mg/kg i.p.) narkotisiert, vagotomiert, mit Atropin (1 mg/kg s.c.) behandelt und trachealkanüliert. Nach Enukleation eines Auges wird die Orbita durchstoßen, das Gehirn zerstört und ein Metallstab durch den Wirbelkanal vorgeschoben. Die Tiere werden künstlich beatmet und mit Gallamin (4 mg/kg i.v.) vorbehandelt. Der verwendete Metallstab hat einen Durchmesser von 2,5 mm und ist isoliert (einbrennemailliert). Vom 7. - 8. cm von der Spitze gemessen ist der Stab blank. Dieses blanke Stück kommt beim Vorschieben des Stabes auf Höhe des letzten Hals- und ersten Brustwirbels zu liegen. Gegen eine Gegenelektrode, die in die Nackenhaut der Tiere eingestochen wird, wird mittels eines Stimulators supramaximal gereizt (Rechteckimpulse 50V, 2 ms; 0,2 Hz; Reizdauer 25 s.

Der Blutdruck wird aus einer A. carotis mittels elektromechanischem Druckwandler gemessen. Die Pulswelle triggert einen Tachographen, der kontinuierlich die Herzfrequenz wiedergibt. Beide Signale werden auf einem Mehrkanalschreiber registriert.

Es wird die Dosis ($D_{50}$) angegeben, welche die elektrisch induzierte Tachykardie um 50 % hemmt.

| Substanz | Hemmung der elektrisch ausgelösten Tachykardie $D_{50}$ |
|---|---|
| A | 290 µg/kg i.v. |

**3. Wirkung an postsynaptischen α-Adrenoceptoren: Blutdrucksteigerung an der Spinalratte:**

**Methodik:**

Am Modell der Spinalratte wird die nach Erregung postsynaptischer α-Adrenorezeptoren eintretende Blutdrucksteigerung gemessen.

**Versuchsbeschreibung:**

Männliche Ratten im Gewicht von 200-250 g werden narkotisiert (1,2 g/kg Urethan i.p., 1 mg/kg Atropin s.c.). Dann erfolgt Eröffnung des Rückenmarkkanals. Anschließend werden bei $C_1$ das Rückenmark durchtrennt und die Medulla oblongata und Gehirn mit einer Metallsonde zerstört. Die Tiere werden künstlich beatmet; in eine Jugularvene wird ein Polyäthylen-Katheter eingebundeh, die Registrierung des Blutdrucks erfolgt aus einer A. carotis mittels Statham-Element an einem Grass-Polygraphen.

Es wird die Dosis ($D_{30}$) angegeben, welche den ar Blutdruck um 30 mmHg steigert.

| Substanz | Blutdrucksteigernde Wirkung $D_{30}$ |
|---|---|
| A | 145 µg/kg i.v. |
| H | 0,36 mg/kg i.v. |

**4. Akute Toxizität:**

Die akute Toxizität der zu untersuchenden Substanzen wurde an Mäusen nach oraler bzw. intravenöser Gabe bestimmt (Beobachtungszeit: 14 Tage):

| Substanz | $LD_{50}$ | |
|---|---|---|
| A | 34 mg/kg i.v. | 320 mg/kg p.o. |
| F | | > 300 mg/kg p.o. |

Auf Grund ihrer pharmakologischen Eigenschaften eignen sich die neuen Verbindungen der allgemeinen Formel I sowie deren physiologisch verträgliche Säureadditionssalze zur Behandlung von Herz- und Kreislauferkrankungen, pectanginösen Beschwerden sowie zur Senkung der Herzfrequenz. Hierzu lassen sich diese, gegebenenfalls in Kombination mit anderen Wirksubstantanzen, in die üblichen galenischen

Zubereitungsformen wie Dragées, Tabletten, Kapseln, Suppositorien oder Lösungen einarbeiten. Die Einzeldosis beträgt 0,02 bis 0,15 mg/kg Körpergewicht, vorzugsweise 0,02 bis 0,08 mg/kg Körpergewicht 1 bis 3 x täglich.

Die nachfolgenden Beispiele sollen die Erfindung näher erläutern:

Herstellung der Ausgangsprodukte:

**Beispiel A**

**2-Amino-5,6,7,8-tetrahydro-4H-thieno[2,3-c]azepin-3,7-dicarbonsäure-diäthylester**

3,7 g (0,02 Mol) Hexahydro-azepinon-(4)-1-carbonsäure-äthylester werden zusammen mit 2,26 g (0,02 Mol) Cyanessigsäureäthylester und 0,65 g (0,02 Mol) Schwefel in 20 ml Äthylalkohol suspendiert. In diese Suspension werden unter Rühren 5 ml Morpholin eingetropft, wobei die Temperatur auf etwa 30°C anstieg. Danach wird noch 3 Stunden bei 50°C weitergerührt und über Nacht bei Raumtemperatur stehen gelassen. Anderntags wird das inzwischen ausgefallene feste Produkt abgesaugt und aus Isopropylalkohol umkristallisiert.

Ausbeute: 3,6 g (58 % der Theorie),

Schmelzpunkt: 104-106°C

| | | | | | | | | |
|---|---|---|---|---|---|---|---|---|
| Ber.: | C | 53,86 | H | 6,41 | N | 8,98 | S | 10,25 |
| Gef.: | | 53,90 | | 6,40 | | 9,04 | | 10,32 |

**Beispiel B**

**2-Amino-5,6,7,8-tetrahydro-4H-thieno[2,3-d]azepin-3-carbonsäuremethylester-hydrochlorid**

Zu einer Lösung von 41 g (0,15 Mol) 5-Brom-hexahydro-azepinon-(4)-hydrobromid in 90 ml Methylalkohol wird bei 10-15°C innerhalb von 25 Minuten eine Lösung von 11,1 g (0,15 Mol) Natriumhydrogensulfid in 120 ml Methylalkohol getropft. Nach weiterem 20-minütigem Rühren bei 10-15°C werden 14,9 g (0,15 Mol) Cyanessigsäuremethylester zugegeben. Unter starkem Rühren werden sodann 21,9 g (0,3 Mol) Diäthylamin innerhalb 20 Minuten zugetropft, wobei die Innentemperatur von 8 auf 26°C ansteigt. Danach wird noch 2 Stunden bei 40°C weitergerührt. Nach Abkühlung wird im Vakuum eingedampft, der Rückstand mit verdünnter Natronlauge aufgenommen und mit Chloroform ausgeschüttelt. Die Chloroformphasen werden mit verdünnter Natronlauge und Wasser gewaschen, über Natriumsulfat getrocknet und im Vakuum eingedampft. Der Rückstand wird an einer Kieselgelsäule (Chloroform/ Methanol/wässr. Ammoniak = 7:4:0,25) chromatographiert. Nach Vereinigung der entsprechenden Fraktionen wird im Vakuum eingedampft. Der Rückstand wird in Methanol aufgenommen und mit isopropylalkoholischer Salzsäure das Hydrochlorid gefällt und abgezangt. Das schwerlösliche Salz wird 2 mal mit Methanol ausgekocht.

Ausbeute: 5,4 g (13,7 % der Theorie),

Schmelzpunkt: Ab 256°C (Zers.)

| | | | | | | | | | | |
|---|---|---|---|---|---|---|---|---|---|---|
| Ber.: | C | 45,71 | H | 5,75 | N | 10,66 | Cl | 13,49 | S | 12,20 |
| Gef.: | | 45,67 | | 5,81 | | 10,64 | | 13,50 | | 12,04 |

**Beispiel C**

**2-(N-tert.-Butyloxycarbonylamino)-6-propyl-5,6,7,8-tetrahydro4H-thieno[2,3-d]azepin**

8,5 g (0,0335 Mol) 5,6,7,8-Tetrahydro-6-propyl-4H-thieno[2,3-d]-azepin-2-carbonsäure-hydrazid werden in 70 ml 2n Salzsäure ge-Jöst und mit einer Lösung von 2,8 g (0,04 Mol) Natriumnitrit in 10 ml Wasser bei -5° bis 0°C diazotiert. Es wird bei 0°C 50 Minuten nachgerührt und der sich dabei bildende dicke Kristallbrei mit 150 ml Methylenchlorid unterschichtet. Danach werden 40 ml konz. Ammoniak-Lösung zugetropft, die Methylenchloridphase abgetrennt und die wässrige Schicht mehrmals mit Methylenchlorid extrahiert. Die vereinigten Methylenchlorid-Phasen werden über Natriumsulfat getrocknet und bei 30°C Badtemperatur am Rotationsverdampfer eingeengt. Danach wird noch 2 mal mit Benzol versetzt und im Vakuum eingedampft.

Ausbeute an Azid: 8,5 g (95,8 % der Theorie), hellbraunes öl.

Die Substanz (0,032 Mol) wird in 25 ml absolutem Dioxan gelöst und innerhalb von 30 Minuten in eine auf 90°C erhitzte Mischung aus 30 ml absolutem Dioxan und 2,9 g (0,0385 Mol) tert.Butylalkohol getropft, wobei eine lebhafte Stickstoffentwicklung auftritt. Danach wird noch weitere 1,5 Stunden zum Sieden erhitzt und über Nacht abkühlen gelassen. Anderntags wird im Vakuum eingedampft und der Rückstand an einer Kieselgelsäule (Essigsäureäthylester/Äthylalkohol/konzentrierter wässriger Ammoniak = 0,2:7:0,7) chromatographiert. Nach Vereinigung der entsprechenden Fraktionen wird im Vakuum eingedampft.

Ausbeute: 5,5 g (55 % der Theorie),

Schmelzpunkt: 141-143°C.

**Beispiel D**

**6-Äthyl-2-amino-5,6,7,8-tetrahydro-4H-thieno[2,3-d]azepin-dihydrobromid**

2,0 g (0,0068 Mol) 6-Äthyl-2-(N-tert.-butyloxycarbonylamino)-5,6,7,8-tetrahydro-4H-thieno[2,3-d]azepin werden unter Einleitung von trockenem Stickstoff unter Rühren und Kühlen auf 0°C in 18 ml einer 40%igen Eisessig-Bromwasserstoff-Lösung eingetragen. Nach 3-stündigem Rühren bei 0°C werden 60 ml absoluten Äthers zugetropft, 15 Minuten gerührt und abdekantiert. Nach mehrmaligem Zusetzen von absolutem Äther und Abdekantieren wird die anfangs klebrige Substanz kristallin und absaugbar. Es wird mehrmals mit absolutem Äther nachgewaschen und im Exsikkator nber Phosphorpentoxyd getrocknet.

Ausbeute: 1,8 g (75 % der Theorie),
Schmelzpunkt: ab 50°C (Zers.).

| | | | | | | | | | | |
|---|---|---|---|---|---|---|---|---|---|---|
| Ber.: | C | 33,54 | H | 5,07 | N | 7,82 | Br | 44,62 | S | 8,95 |
| Gef.: | | 33,80 | | 5,29 | | 7,36 | | 44,30 | | 8,84 |

Analog den Beispielen A bis D wurden folgende Verbindungen hergestellt:

2-Amino-6-benzyl-5,6,7,8-tetrahydro-4H-thieno [2,3-d] azepin-3-carbonsäure-äthylester-hydrochlorid
Ausbeute: 23 % der Theorie,
Schmelzpunkt: 228-229°C (Zers.)

| | | | | | | | | | | |
|---|---|---|---|---|---|---|---|---|---|---|
| Ber.: | C | 58,93 | H | 6,32 | N | 7,63 | Cl | 9,66 | S | 8,74 |
| Gef.: | | 58,64 | | 6,32 | | 7,36 | | 9,34 | | 8,52 |

6-Äthyl-2-amino-5,6,7,8-tetrahydro-4H-thieno [2,3-d] azepin-3-carbonsäure-äthylester-oxalat
Ausbeute: 32 % der Theorie,
Schmelzpunkt: 173-174°C (Zers.)

| | | | | | | | | |
|---|---|---|---|---|---|---|---|---|
| Ber.: | C | 50,27 | H | 6,19 | N | 7,82 | S | 8,94 |
| Gef.: | | 50,25 | | 6,36 | | 7,64 | | 9,23 |

6-Allyl-2-amino-5,6,7,8-tetrahydro-4H-thieno [2,3-d] azepin-3-carbonsäure-äthylester
Ausbeute: 26,1 % der Theorie, rotes öl

Ber.: Molpeak m/e = 280
Gef.: Molpeak m/e = 280

2-Amino-5,6,7,8-tetrahydro-4H-thieno [2,3-d] azepin-3,6-dicarbon-säure-diäthylester und
2-Amino-5,6,7,8-tetrahydro-4H-thieno[2,3-c] azepin-3,7-dicarbon-säure-diäthylester
Ausbeute: 16,1 % der Theorie,
Schmelzpunkt: 122-125°C

| | | | | | | | | |
|---|---|---|---|---|---|---|---|---|
| Ber.: | C | 53,83 | H | 6,45 | N | 8,97 | S | 10,26 |
| Gef.: | | 54,00 | | 6,35 | | 9,16 | | 10,06 |

2-(N-tert.-Butyloxycarbonylamino)-5,6,7,8-tetrahydro-6-isopro-pyl-4H-thieno [2,3-d] azepin
Ausbeute: 38,5 % der Theorie

| | | | | | | | | |
|---|---|---|---|---|---|---|---|---|
| Ber.: | C | 61,90 | H | 8,44 | N | 9,02 | S | 10,33 |
| Gef.: | | 61,68 | | 8,56 | | 9,18 | | 10,19 |

2-Amino-5,6,7,8-tetrahydro-6-isopropyl-4H-thieno [2,3-d] azepindihydrobromid
Ausbeute: 83,5 % der Theorie

| | | | | | | | | | | |
|---|---|---|---|---|---|---|---|---|---|---|
| Ber.: | C | 35,50 | H | 5,42 | N | 7,53 | S | 8,61 | Br | 42,94 |
| Gef.: | | 35,19 | | 5,63 | | 7,64 | | 8,32 | | 42,51 |

6-Allyl-2-(N-tert.-butyloxycarbonylamino)-5,6,7,8-tetrahydro-4H-thieno [2,3-d] azepin
Ausbeute: 29,6 % der Theorie,
Schmelzpunkt: 135-137°C

| | | | | | | | | |
|---|---|---|---|---|---|---|---|---|
| Ber.: | C | 62,31 | H | 7,84 | N | 9,08 | S | 10,39 |
| Gef.: | | 62,40 | | 7,93 | | 9,06 | | 10,50 |

6-Allyl-2-amino-5,6,7,8-tetrahydro-4H-thieno [2,3-d] azepin-dihydrobromid
Ausbeute: 83,3 % der Theorie,
Schmelzpunkt: ab 50°C (Zers.).

| | | | | | | | | | | |
|---|---|---|---|---|---|---|---|---|---|---|
| Ber.: | C | 35,69 | H | 4,90 | N | 7,57 | Br | 43,18 | S | 8,66 |
| Gef.: | | 35,70 | | 5,33 | | 6,95 | | 42,70 | | 8,36 |

2-Amino-6-(4-chlor-benzyl)-5,6,7,8-tetrahydro-4H-thieno [2,3-d]-azepin-dihydrobromid
Ausbeute: 75 % der Theorie,
Schmelzpunkt: ab 225°C (Zers.).

Ber.: Molpeak m/e = 292/294 (1 Cl)

Gef.: Molpeak m/e = 292/294 (1 Cl)

6-Benzyl-2-(N-tert.-butyloxycarbonylamino)-5,6,7,8-tetrahydro-4H-thieno [2,3-d] azepin
Ausbeute: 45 % der Theorie, gelbes Öl

Ber.: Molpeak m/e = 358

Gef.: Molpeak m/e = 358

2-Amino-6-benzyl-5,6,7,8-tetrahydro-4H-thieno [2,3-d] azepin-dihydrobromid
Ausbeute: 84 % der Theorie,
Schmelzpunkt: 232-234°C

| | | | | | | | | | | |
|---|---|---|---|---|---|---|---|---|---|---|
| Ber.: | C | 42,87 | H | 4,80 | N | 6,67 | Br | 38,03 | S | 7,63 |
| Gef.: | | 42,90 | | 4,87 | | 6,37 | | 37,90 | | 7,80 |

6-Äthyl-2-(N-tert.-butyloxycarbonylamino)-5,6,7,8-tetrahydro-4H-thieno [2,3-d] azepin
Ausbeute: 30,5 % der Theorie,
Schmelzpunkt: 149-151°C

| | | | | | | | | |
|---|---|---|---|---|---|---|---|---|
| Ber.: | C | 60,78 | H | 8,16 | N | 9,45 | S | 10,82 |
| Gef.: | | 61,00 | | 8,22 | | 9,40 | | 11,04 |

5-Äthyl-2-(N-tert.-butyloxycarbonylamino)-5,6,7,8-tetrahydro-4H-thieno [3,2-c] azepin
Ausbeute: 67,5 % der Theorie,
Schmelzpunkt: 154-156°C
5-Äthyl-2-amino-5,6,7,8-tetrahydro-4H-thieno [3,2-c] azepin-dihydrobromid
Ausbeute: 100 % der Theorie (hygroskopisch)
Schmelzpunkt: Sintern ab 100°C, ab 210° (Zers.).
Herstellung der Endprodukte:

**Beispiel 1**

**7-Äthyl-2-amino-6,7,8,9-tetrahydro-5H-thiazolo [4',5':5,4] thieno[2,3-d] azepin-dihydrochlorid**

16,9 g (0,063 Mol) 6-Äthyl-2-amino-5,6,7,8-tetrahydro-4H-thieno-[2,3-d] azepin-dihydrochlorid werden in 200 ml Eisessig unter Zusatz von 10 ml Wasser bis zur vollständigen Lösung gerührt. Danach wird mit einer Lösung von 36,6 g (0,37 Mol) Kaliumrhodanid in 40 ml Wasser versetzt und eine Lösung von 10,0 g Brom (0,063 Mol) in 30 ml Eisessig langsam zugetropft. Nach Abklingen der Reaktion wird eine Stunde nachgerührt und über Nacht stehen gelassen. Nach Filtration vom Unlöslichen wird im Vakuum eingeengt, der schwarze Rückstand mehrmals mit warmem Wasser digeriert und jeweils über Celite abgesaugt. Die vereinigten wässrigen Filtrate werden mit konzentriertem Ammoniak alkalisch gestellt, in Eiswasser gekühlt, das ausgefallene Festprodukt abgesaugt und 2 mal mit kaltem Wasser gewaschen. Nach dem Trocknen wird die Substanz 3 Stunden in Chloroform bei Raumtemperatur gerührt, abgesaugt, mit kaltem Chloroform gewaschen und getrocknet.

Die hellbraunen Kristalle werden in heißem Methanol unter Zusatz von etwas Chloroform gelöst und mit äthanolischer Salzsäure angesäuert. Nach Abkühlen in Eiswasser wird das Dihydrochlorid abgesaugt und getrocknet.

Ausbeute: 6 g (30 % der Theorie),
Schmelzpunkt: 256°C (Zers.)

| | | | | | | | | | | |
|---|---|---|---|---|---|---|---|---|---|---|
| Ber.: | C | 40,49 | H | 5,25 | Cl | 21,73 | N | 12,88 | S | 19,65 |
| Gef.: | | 40,60 | | 5,12 | | 21,55 | | 13,01 | | 19,50 |

**Beispiel 2**

**2-Amino-7-benzyl-6,7,8,9-tetrahydro-5H-thiazolo [4',5':5,4]thieno [2,3-d] azepin**

Hergestellt aus 2-Amino-6-benzyl-5,6,7,8-tetrahydro-4H-thieno-[2,3-d] azepin-dihydrochlorid, Kaliumrhodanid und Brom analog Beispiel 1.

Ausbeute: 56 % der Theorie,
Schmelzpunkt: 191-192°C

| | | | | | | | | |
|---|---|---|---|---|---|---|---|---|
| Ber.: | C | 60,92 | H | 5,43 | N | 13,32 | S | 20,33 |
| Gef.: | | 61,24 | | 5,53 | | 12,97 | | 20,45 |

**Beispiel 3**

**2-Amino-7-(4-chlorbenzyl)-6,7,8,9-tetrahydro-5H-thiazolo[4',5':5,4] thieno [2,3-d] azepin-dihydrochlorid**

Hergestellt aus 2-Amino-6-(4-chlorbenzyl)-5,6,7,8-tetrahydro-4H-thieno [2,3-d] azepin-dihydrochlorid, Kaliumrhodanid und Brom analog Beispiel 1.
Ausbeute: 12 % der Theorie,
Schmelzpunkt: 220° C (Zers.)

| | | | | | | | | | | |
|---|---|---|---|---|---|---|---|---|---|---|
| Ber.: | C | 45,45 | H | 4,29 | Cl | 25,15 | N | 9,94 | S | 15,17 |
| Gef.: | | 45,60 | | 4,15 | | 25,10 | | 10,02 | | 15,35 |

**Beispiel 4**

**2-Amino-7-propyl-6,7,8,9-tetrahydro-5H-thiazolo [4',5:5,4]thieno [2,3-d] azepin-dihydrochlorid**

Hergestellt aus 2-Amino-6-propyl-5,6,7,8-tetrahydro-4H-thieno [2,3-d] azepin-dihydrochlorid, Kaliumrhodanid und Brom analog Beispiel 1.
Ausbeute: 22 % der Theorie,
Schmelzpunkt: 230-233° C (Zers.)

| | | | | | | | | | | |
|---|---|---|---|---|---|---|---|---|---|---|
| Ber.: | C | 42,35 | H | 5,63 | Cl | 20,83 | N | 12,35 | S | 18,84 |
| Gef.: | | 42,25 | | 5,79 | | 20,45 | | 12,50 | | 18,95 |

**Beispiel 5**

**2=Amino-7-isopropyl-6,7,8,9-tetrahydro-5H-thiazolo [4',5:5,4]thieno [2,3-d] azepin-dihydrochlorid**

Hergestellt aus 2-Amino-5,6,7,8-tetrahydro-6-isopropyl-4H-thieno [2,3-d] azepin-dihydrochlorid, Kaliumrhodanid und Brom analog Beispiel 1.
Ausbeute: 28 % der Theorie,
Schmelzpunkt: 243-245° C (Zers.)

| | | | | | | | | | | |
|---|---|---|---|---|---|---|---|---|---|---|
| Ber.: | C | 42,35 | H | 5,63 | Cl | 20,83 | N | 12,35 | S | 18,84 |
| Gef.: | | 42,35 | | 6,00 | | 21,00 | | 12,27 | | 18,85 |

**Beispiel 6**

7-Äthyl-2-amino-5,6,7,8-tetrahydro-thiazolo [4',5':5,4] thieno-[3,2-c] pyridin-dihidrochlorid
Hergestellt aus 2-Amino-5-äthyl-4,5,6,7-tetrahydro-thieno-[3,2-c] pyridin, Kaliumrhodanid und Brom analog Beispiel 1.
Ausbeute: 24 % der Theorie,
Schmelzpunkt: > 250° C

| | | | | | | | | | | |
|---|---|---|---|---|---|---|---|---|---|---|
| Ber.: | C | 38,46 | H | 4,84 | Cl | 22,71 | N | 13,46 | S | 20,53 |
| Gef.: | | 38,68 | | 4,90 | | 22,52 | | 13,41 | | 20,30 |

**Beispiel 7**

**2-Amino-7-propyl-5,6,7,8-tetrahydro-thiazolo [4',5':5,4] thieno [3,2-c] pyridin-dihydrochlorid**

Hergestellt aus 2-Amino-5-propyl-4,5,6,7-tetrahydro-thieno-[3,2-c] pyridin, Kaliumrhodanid und Brom analog Beispiel 1.
Ausbeute: 29 % der Theorie,
Schmelzpunkt: 252-255° C (Zers.)

| | | | | | | | | | | |
|---|---|---|---|---|---|---|---|---|---|---|
| Ber.: | C | 40,49 | H | 5,25 | Cl | 21,73 | N | 12,88 | S | 19,65 |
| Gef.: | | 40,45 | | 5,30 | | 21,70 | | 13,02 | | 19,65 |

**Beispiel 8**

**2-Amino-7-isopropyl-5,6,7,8-tetrahydro-thiazolo [4,5':5,4]thieno [3,2-c] pyridin-dihydrochlorid**

Hergestellt aus 2-Amino-5-isopropyl-4,5,6,7-tetrahydro-thieno-[3,2-c] pyridin, Kaliumrhodanid und Brom analog Beispiel 1.
Ausbeute: 23 % der Theorie,
Schmelzpunkt: 271-273° C (Zers.)

| | | | | | | | | | | |
|---|---|---|---|---|---|---|---|---|---|---|
| Ber.: | C | 40,49 | H | 5,25 | Cl | 21,73 | N | 12,88 | S | 19,65 |
| Gef.: | | 40,71 | | 5,21 | | 21,72 | | 12,91 | | 19,43 |

**Beispiel 9**

**7-Allyl-2-amino-5,6,7,8-tetrahydro-thiazolo [4',5':5,4] thieno [3,2-c] pyridin-dihydrochlorid**

Hergestellt aus 5-Allyl-2-amino-4,5,6,7-tetrahydro-thieno-[3,2-c] pyridin, Kaliumrhodanid und Brom analog Beispiel 1.
Ausbeute: 22 % der Theorie,
Schmelzpunkt: 246-247° C (Zers.)

| | | | | | | | | | | |
|---|---|---|---|---|---|---|---|---|---|---|
| Ber.: | C | 40,74 | H | 4,66 | Cl | 21,87 | N | 12,96 | S | 19,77 |
| Gef.: | | 40,52 | | 4,90 | | 21,80 | | 13,06 | | 19,77 |

**Beispiel 10**

**2-Amino-6,7,8,9-tetrahydro-5h-thiazolo [4',5':5,4] thieno [2,3-d]azepin-7-carbonsäureäthylester-hydrochlorid**

7,6 g (0,025 Mol) 2-Amino-6-carbäthoxy-5,6,7,8-tetrahydro-4H-thieno [2,3-d] azepin-3-carbonsäure-Natriumsalz (hergestellt durch Verseifung von 7,8 g (0,025 Mol) 2-Amino-5,6,7,8-tetrahydro-4H-thieno [2,3-d] azepin-3,6-dicarbonsäure-diäthylester mit Natronlauge) werden in 125 ml Eisessig gelöst und 7,3 g (0,075 Mol) Kaliumrhodanid und 12,5 ml Wasser zugegeben. Die Reaktionsmischung wird unter Rühren tropfenweise mit einer Lösung von 4 g (0,025 Mol) Brom in 17 ml Eisessig versetzt und noch 3 Stunden bei Raumtemperatur weitergerührt. Anschließend wird in 500 ml Wasser gegossen, mit konzentriertem wässrigem Ammoniak alkalisch gemacht und 5 mal mit Chloroform extrahiert. Die vereinigten Chloroformextrakte werden 3-4 mal mit Wasser gewaschen, mit Natriumsulfat und Kaliumkarbonat getrocknet und im Vakuum eingedampft. Der feste Rückstand wird 2 mal an einer Kieselgelsäule (150 ml; Chloroform/Methanol = 100:3 und Chloroform/Essigsäureäthylester = 17:3) chromatographisch gereinigt. Nach Eindampfen der entsprechenden Fraktionen im Vakuum wird der Rückstand in einem Chloroform/Methanol-Gemisch = 10:1 gelöst und mit isopropanolischer Salzsäure angesäuert. Hierauf wird das Reaktionsgemisch nochmals im Vakuum eingedampft, der Rückstand in Essigsäureäthylester aufgekocht und nach Abkühlen in Eiswasser abgesaugt.
Ausbeute: 1,7 g (20,4 % der Theorie),
Schmelzpunkt: 212° C (Zers.)

| | | | | | | | | | | |
|---|---|---|---|---|---|---|---|---|---|---|
| Ber.: | C | 43,17 | H | 4,83 | Cl | 10,62 | N | 12,59 | S | 19,21 |
| Gef.: | | 43,31 | | 5,01 | | 10,75 | | 12,79 | | 19,45 |

**Beispiel 11**

**2-Amino-6,7,8,9-tetrahydro-5H-thiazolo [4',5':5,4] thieno [2,3-d]azepin-dihydrochlorid**

Hergestellt aus 2-Amino-5,6,7,8-tetrahydro-4H-thieno [2,3-d] aze-pin-3-carbonsäure-Natriumsalz, Kaliumrhodanid und Brom analog Beispiel 10.
Ausbeute: 22,3 % der Theorie,
Schmelzpunkt: > 310° C

| | | | | | | | | | | |
|---|---|---|---|---|---|---|---|---|---|---|
| Ber.: | C | 36,24 | H | 4,39 | Cl | 23,77 | N | 14,09 | S | 21,50 |
| Gef.: | | 36,01 | | 4,58 | | 23,60 | | 13,75 | | 21,30 |

**Beispiel 12**

**7-Allyl-2-amino-6,7,8,9-tetrahydro-5H-thiazolo [4',5':5,4] thieno[2,3-d] azepin-dihydrochlorid**

Hergestellt aus 6-Allyl-2-amino-5,6,7,8-tetrahydro-4H-thieno-[2,3-d] azepin-3-carbonsäure-Natriumsalz, Kaliumrhodanid und Brom analog Beispiel 10.
Ausbeute: 15 % der Theorie,
Schmelzpunkt: 232-233° C (Zers.)

| | | | | | | | | | | |
|---|---|---|---|---|---|---|---|---|---|---|
| Ber.: | C | 42,60 | H | 5,06 | Cl | 20,96 | N | 12,42 | S | 18,95 |
| Gef.: | | 42,89 | | 4,96 | | 20,80 | | 12,78 | | 18,80 |

**Beispiel 13**

**2-Amino-5,6,7,8-tetrahydro-6-isopropyl-thiazolo [4',5':5,4] thieno[2,3-c]pyridin-dihydrochlorid**

Hergestellt aus 2-Amino-6-isopropyl-4,5,6,7-tetrahydro-thieno-[2,3-c] pyridin-3-carbonsäure-Natriumsalz, Kaliumrhodanid und Brom analog Beispiel 10.
Ausbeute: 24,5 % der Theorie,
Schmelzpunkt: 250° C (Zers.)

| | | | | | | | | | | |
|---|---|---|---|---|---|---|---|---|---|---|
| Ber.: | C | 40,49 | H | 5,25 | Cl | 21,73 | N | 12,88 | S | 19,65 |
| Gef.: | | 40,54 | | 5,26 | | 21,80 | | 13,08 | | 19,45 |

**Beispiel 14**

**6-Allyl-2-amino-5,6,7,8-tetrahydro-thiazolo [4',5':5,4] thieno[2,3-c] pyridin-dihydrochlorid**

Hergestellt aus 6-Allyl-2-amino-4,5,6,7-tetrahydro-thieno [2,3-c]-pyridin-3-carbonsäure-Natriumsalz, Kaliumrhodanid und Brom analog Beispiel 10.
Ausbeute: 7 % der Theorie,
Schmelzpunkt: 165° C (Zers.)

| | | | | | | | | | | |
|---|---|---|---|---|---|---|---|---|---|---|
| Ber.: | C | 40,74 | H | 4,66 | Cl | 21,87 | N | 12,96 | S | 19,77 |
| Gef.: | | 40,60 | | 4,79 | | 21,88 | | 12,96 | | 19,70 |

**Beispiel 15**

**6-Äthyl-2-amino-5,6,7,8-tetrahydro-thiazolo [4',5':5,4] thieno[2,3-c] pyridin-dihydrochlorid**

Hergestellt aus 6-Äthyl-2-amino-4,5,6,7-tetrahydro-thieno-[2,3-c] pyridin-3-carbonsäure-Natriumsalz, Kaliumrhodanid und Brom analog Beispiel 10.
Ausbeute: 12 % der Theorie,
Schmelzpunkt: 237° C (Zers.)

| | | | | | | | | | | |
|---|---|---|---|---|---|---|---|---|---|---|
| Ber.: | C | 38,46 | H | 4,84 | Cl | 22,71 | N | 13,46 | S | 20,54 |
| Gef.: | | 38,25 | | 4,84 | | 22,40 | | 13,40 | | 20,20 |

**Beispiel 16**

**2-Amino-6-propyl-5,6,7,8-tetrahydro-thiazolo [4',5':5,4] thieno[2,3-c] pyridin-dihydrochlorid**

Hergestellt aus 2-Amino-6-propyl-4,5,6,7-tetrahydro-thieno-[2,3-c] pyridin-3-carbonsäure Natriumsalz, Kaliumrhodanid und Brom analog Beispiel 10.
Ausbeute: 12 % der Theorie,
Schmelzpunkt: 224° C (Zers.)

| | | | | | | | | | | |
|---|---|---|---|---|---|---|---|---|---|---|
| Ber.: | C | 40,49 | H | 5,25 | Cl | 21,73 | N | 12,88 | S | 19,65 |
| Gef.: | | 40,64 | | 5,41 | | 21,54 | | 13,28 | | 19,90 |

**Beispiel 17**

**2-Amino-6-methyl-5,6,7,8-tetrahydro-thiazolo [4',5':5,4] thieno[2,3-c] pyridin-dihydrochlorid**

Hergestellt aus 2-Amino-6-methyl-4,5,6,7-tetrahydro-thieno-[2,3-c] pyridin-3-carbonsäure-Natriumsalz, Kaliumrhodanid und Brom analog Beispiel 10.
Ausbeute: 4 % der Theorie,
Schmelzpunkt: 260-262° C (Zers.)

| | | | | | | | | | | |
|---|---|---|---|---|---|---|---|---|---|---|
| Ber.: | C | 36,24 | H | 4,39 | Cl | 23,77 | N | 14,09 | S | 21,50 |
| Gef.: | | 36,15 | | 4,37 | | 23,65 | | 13,96 | | 21,40 |

**Beispiel 18**

**6-Acetyl-2-amino-5,6,7,8-tetrahydro-thiazolo [4',5':5,4] thieno[2,3-c] pyridin-hydrochlorid**

Hergestellt aus 6-Acetyl-2-amino-4,5,6,7-tetrahydro-thieno-[2,3-c] pyridin-3-carbonsäure-Natriumsalz, Kaliumrhodanid und Brom analog Beispiel 10.
Ausbeute: 5 % der Theorie,
Schmelzpunkt: 215° C (Zers.)

| | | | | | | | | | | |
|---|---|---|---|---|---|---|---|---|---|---|
| Ber.: | C | 41,44 | H | 4,17 | Cl | 12,23 | N | 14,50 | S | 22,13 |
| Gef.: | | 41,77 | | 3,98 | | 12,42 | | 14,50 | | 22,00 |

**Beispiel 19**

**2-Amino-7-methyl-5,6,7,8-tetrahydro-thiazolo [4',5':5,4] thieno[3,2-c] pyridin-dihydrochlorid**

Hergestellt aus 2-Amino-6-methyl-4,5,6,7-tetrahydro-thieno-[3,2-c] pyridin-3-carbonsäure-Natriumsalz, Kaliumrhodanid und Brom analog Beispiel 10.
Ausbeute: 5,4 % der Theorie,
Schmelzpunkt: 259-260° C (Zers.)

Ber.: Molpeak m/e = 225
Gef.: Molpeak m/e = 225

**Beispiel 20**

**2-Amino-6,7,8,9-tetrahydro-7-methyl-5H-thiazolo [4',5':5,4] thieno[2,3-d] azepin-dihydrochlorid**

3 g (0,01 Mol) 2-Amino-6,7,8,9-tetrahydro-5H-thiazolo [4',5:5,4]-thieno [2,3-d] azepin-7-carbonsäureäthylester werden in 30 ml absolutem Tetrahydrofuran gelöst und bei Raumtemperatur langsam unter Rühren in eine Suspension von 3g (0,08 Mol) Lithiumaluminiumhydrid in 50 ml absolutem Tetrahydrofuran getropft. Die Temperatur der Reaktionsmischung wird dabei durch gelegentliches Kühlen in Eiswasser unter 30° C gehalten. Man rührt weitere 6 Stunden bei Raumtemperatur und läßt über Nacht stehen. Anderntags werden unter Eiskühlung langsam 18 ml Essigsäureäthylester zugetropft, sodaß die Temperatur unterhalb 25° C gehalten wird. Schließlich gibt man vorsichtig 10 ml Wasser und 3 ml 10%ige Natronlauge zu. Der Hydroxydniederschlag wird über Celite abgesaugt und mit heißem Chloroform]Methanolgemisch bzw. Chloroform mehrmals gewaschen. Nach Eindampfen des Filtrats hinterbleiben 3 g eines viskosen, dunklen Öls, das durch Säulenchromatographie an Kieselgel [Chloroform/Methanol/konzentrierter wässriger Ammoniak = 8,5:1,5:0,15] gereinigt wird. Nach Eindampfen der entsprechenden Fraktionen wird der Rückstand in Isopropanol gelöst und mit isopropanolischer Salzsäure angesäuert. Das ausgefallene Dihydrochlorid wird abgesaugt und mit kaltem Isopropanol gewaschen.
Ausbeute: 0,55 g (18 % der Theorie),
Schmelzpunkt: 271-273° C (Zers.)

| | | | | | | | | | | |
|---|---|---|---|---|---|---|---|---|---|---|
| Ber.: | C | 38,46 | H | 4,84 | Cl | 22,71 | N | 13,46 | S | 20,53 |
| Gef.: | | 38,80 | | 4,50 | | 22,90 | | 13,37 | | 20,55 |

**Beispiel 21**

**2-Amino-6,7,8,9-tetrahydro-6-methyl-5H-thiazolo [4',5':5,4] thieno[2,3-c] azepin-dihydrochlorid**

Hergestellt durch Reduktion von 2-Amino-6-carbäthoxy-6,7,8,9-tetrahydro-5H-thiazolo [4',5':5,4] thieno [2,3-c] azepin mit Lithiumaluminiumhydrid analog Beispiel 20.
Ausbeute: 26 % der Theorie,
Schmelzpunkt: 217-220° C (Zers.)

| | | | | | | | | | | |
|---|---|---|---|---|---|---|---|---|---|---|
| Ber.: | C | 38,46 | H | 4,84 | Cl | 22,71 | N | 13,46 | S | 20,53 |
| Gef.: | | 38,25 | | .4,99 | | 22,50 | | 13,54 | | 20,25 |

**Beispiel 22**

**6-Äthyl-2-amino-5,6,7,8-tetrahydro-thiazolo [4',5':5,4] thieno[2,3-c-]pyridin-dihydrochlorid**

Hergestellt durch Reduktion von 6-Acetyl-2-amino-5,6,7,8-tetra-hydro-thiazolo [4',5':5,4] thieno [2,3-c] pyridin mit Lithiumaluminiumhydrid analog Beispiel 20.
Ausbeute: 29 % der Theorie,
Schmelzpunkt: 237° C (Zers.)

Ber.: Molpeak m/e = 239
Gef.: Molpeak m/e = 239

**Beispiel 23**

**8-Äthyl-2-amino-6,7,8,9-tetrahydro-5H-thiazolo [4',5:5,4] thieno [3,2-c] azepin-dihydrochlorid**

Hergestellt aus 5-Äthyl-2-amino-5,6,7,8-tetrahydro-4H-thieno-[3,2-c] azepin-dihydrobromid, Kaliumrhodanid und Brom analog Beispiel 1.
Ausbeute: 21 % der Theorie,
Schmelzpunkt: 241-242° C (Zers.)

| | | | | | | | | | | |
|---|---|---|---|---|---|---|---|---|---|---|
| Ber.: | C | 40,49 | H | 5,25 | Cl | 21,73 | N | 12,88 | S | 19,65 |
| Gef.: | | 40,97 | | 5,34 | | 22,05 | | 13,07 | | 20,15 |

**Beispiel 24**

**2-Amino-7-(2-chlorbcnzyl)-6,7,8,9-tetrahydro-5H-thiazolo[4',:5,4] thieno- [2,3-d] azepin-dihydrochlorid**

Hergestellt aus 2-Amino-6-(2-chlorbenzyl)-5,6,7,8-tetrahydro-4H-thieno[2,3-d]azepin-dihydrochlorid, Baliumrhodamid und Brom oder Kupfersulfat als Oxidasionsmittel analog Beispiel 1.
Ausbeute: 18 % der Theorie,
Schmelzpunkt: 203-206° C (Zers.)

| | | | | | | | | |
|---|---|---|---|---|---|---|---|---|
| Ber.: | C | 45,45 | H | 4,29 | N | 9,94 | S | 15,17 |
| Gef.: | | 45,21 | | 4,17 | | 9,83 | | 15,15 |

**Beispiel I**

**Dragéekern mit 5 mg 7-Äthyl-2-amino-6,7,8,9-tetrahydro-5H-thiazolo[4',5':5,4] thieno [2,3-d] azepin-dihydrochlorid**

Zusammensetzung:
1 Dragéekern enthält:

| | |
|---|---|
| Wirksubstanz | 5,0 mg |
| Milchzucker | 33,5 mg |
| Maisstärke | 10,0 mg |
| Gelatine | 1,0 mg |
| Magnesiumstearat | 0,5 mg |
| | 50,0 mg |

**Herstellungsverfahren:**

Die Mischung der Wirksubstanz mit Milchzucker und Maisstärke wird mit einer 10%igen wäßrigen Gelatinelösung durch Sieb 1 mm granuliert, bei 40°C getrocknet und nochmals durch obiges Sieb gerieben. Das so erhaltene Granulat wird mit Magnesiumstearat gemischt und zu Dragéekernen verpreßt. Die Herstellung muß in abgedunkelten Räumen vorgenommen werden.

Kerngewicht: 50 mg

Stempel: 5 mm, gewölbt

Die so erhaltenen Dragéekerne werden nach bekanntem Verfahren mit einer Hülle überzogen, die im wesentlichen aus Zucker und Talkum besteht. Die fertigen Dragées werden mit Hilfe von Bienenwachs poliert.

Dragéegewicht: 100 mg

**Beispiel II**

**Tropfen mit 5 mg 7-Äthyl-2-amino-6,7,8,9-tetrahydro-5H-thiazolo[4',5':5,4] thieno [2,3-d] azepin-dihydrochlorid**

Zusammensetzung:

100 ml Tropflösung enthalten:

| | | | |
|---|---|---|---|
| p-Oxybenzoesäuremethylester | | 0,035 | g |
| p-Oxybenzoesäurepropylester | | 0,015 | g |
| Anisöl | | 0,05 | g |
| Menthol | | 0,06 | g |
| Äthanol rein | | 10,0 | g |
| Wirksubstanz | | 0,5 | g |
| Zitronensäure | | 0,7 | g |
| Natriumphosphat sek. . 2 $H_2O$ | | 0,3 | g |
| Natriumcyclamat | | 1,0 | g |
| Glycerin | | 15,0 | g |
| Dest. Wasser | ad | 100,0 | ml |

**Herstellungsverfahren:**

Die p-Oxybenzoesäureester, Anisöl sowie Menthol werden in Äthanol gelöst (Lösung I).

Mie Puffersubstanzen, die Wirksubstanz und Natriumcyclamat werden in dest. Wasser gelöst und Glycerin zugefügt (Lösung II). Lösung I wird in Lösung II eingerührt und die Mischung mit dest. Wasser auf das gegebene Volumen aufgefüllt. Die fertige Tropflösung wird durch ein geeignetes Filter filtriert. Die Herstellung und Abfüllung der Tropflösung muß unter Lichtschutz und unter Schutzbegasung erfolgen.

**Beispiel III**

**Suppositorien mit 10 mg 7-Äthyl-2-amino-6,7,8,9-tetrahydro-5Hthiazolo[4',5':5,4]thieno[2,3-d]azepin-dihydrochlorid**

1 Zäpfchen enthält:

| | |
|---|---|
| Wirksubstanz | 10,0 mg |
| Zäpfchenmassen (z.B. Witepsol W 45) | 1 690,0 mg |
| | 1 700,0 mg |

**Herstellungsverfahren:**

Die feingepulverte Substanz wird mit Hilfe eines Eintauchhomogenisators in die geschmolzene und auf 40°C abgekühlte Zäpfchenmasse eingerührt. Die Masse wird bei 35°C in leicht vorgekühlte Formen ausgegossen.
Zäpfchengewicht: 1,7 g

**Beispiel IV**

**Ampullen mit 5 mg 7-Äthyl-2-amino-6,7,8,9-tetrahydro-5H-thiazolo [4',5':5,4] thieno [2,3-d] azepin-dihydrochlorid**

1 Ampulle enthält:

| | | |
|---|---|---|
| Wirksubstanz | | 5,0 mg |
| Zitronensäure | | 7,0 mg |
| Natriumphosphat sek. . 2 $H_2O$ | | 3,0 mg |
| Natriumpyrosulfit | | 1,0 mg |
| Dest. Wasser | ad | 1,0 ml |

**Herstellungsverfahren:**

In ausgekochtem und unter $CO_2$-Begasung abgekühltem Wasser werden nacheinander die Puffersubstanzen, die Wirksubstanz sowie Natriumpyrosulfit gelöst. Man füllt mit abgekochtem Wasser auf das gegebene Volumen auf und filtriert Pyrogenfrei.
Abfüllung: in braune Ampullen unter Schutzbegasung
Sterilisation: 20 Minuten bei 120°C
Die Herstellung und Abfüllung der Ampullenlösung muß in abgedunkelten Räumen vorgenommen werden.

**Beispiel V**

**Dragées mit 1 mg 7-Äthyl-2-amino-6,7,8,9-tetrahydro-5H-thiazolo[4',5':5-4] thieno [2,3-d] azepin-dihydrochlorid**

1 Drageékern enthält:

| | |
|---|---|
| Wirksubstanz | 1,0 mg |
| Milchzucker | 35,5 mg |
| Maisstärke | 12,0 mg |
| Gelatine | 1,0 mg |
| Magnesiumstearat | 0,5 mg |
| | 50,0 mg |

**Herstellungsverfahren:**

Analog Beispiel I.

| | |
|---|---|
| Kerngewicht: | 50 mg |
| Stempel: | 5 mm, gewölbt |
| Drageégewicht: | 100 mg |

**Patentansprüche** für die Vertragsstaaten BE CH DE FR IT LI LU NL SE

1. Thieno-thiazol-Derivate der allgemeinen Formel

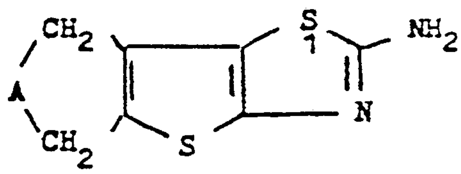

,(I)

in der
A eine Gruppe der Formel

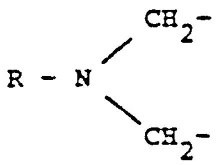
,
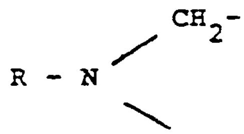
oder
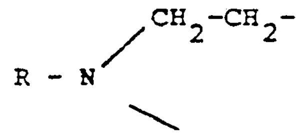

darstellt, wobei
R ein Wasserstoffatom, eine Alkyl- oder Alkanoylgruppe mit 1 bis 5 Kohlenstoffatomen, die Allyl-, Crotyl-, Penten-2 bzw. -3-ylgruppe, eine Alkoxycarbonylgruppe mit insgesamt 2 bis 5 Kohlenstoffatomen oder eine gegebenenfalls durch ein Halogenatom substituierte Aralkylgruppe mit 7 bis 11 Kohlenstoffatomen bedeutet, und deren Säureadditionssalze.

2. 2-Amino-6,7,8,9-tetrahydro-5H-thiazolo [4',5':5,4] thieno-[2,3-d] azepin-, 2-Amino-5,6,7,8-tetrahydro-thiazolo-[4',5':5,4] thieno [3,2-c] pyridin-, 2-Amino-5,6,7,8-tetrahy-dro-thiazolo [4',5':5,4] thieno [2,3-c] pyridin und 2-Amino-6,7,8,9-tetrahydro-5H-thiazolo [4',5':5,4] thieno [2,3-c] azepinund 2-Amino-6,7,8,9-tetrahydro-5H-thiazolo [4',5':5,4] thi [3,2-c] azepin-Derivate der allgemeinen Formel I gemäß Anspruch 1, in der
R ein Wasserstoffatom, eine Alkylgruppe mit 1 bis 4 Kohlen stoffatomen, eine Alkanoylgruppe mit 1 bis 3 Kohlenstoff atomen, eine Alkoxycarbonylgruppe mit insgesamt 2 bis 4 Koblenstoffatomen, eine gegebenenfalls durch ein Chloroder Bromatom substituiezte Benzylgruppe oder eine Allylgruppe darstellt, und deren Säureadditionssalze.

3. 2-Amino-6,7,8,9-tetrahydro-5H-thiazolo [4',5':5,4] thieno-[2,3-d] azepin- und 2-Amino-5,6,7,8-tetrahydro-thiazolo-[4',5':5,4] thieno [2,3-c] pyridin-Derivate der allgemeinen Formel I gemäß Anspruch 1, in der
R ein Wasserstoffatom, eine Alkylgruppe mit 1 bis 3 Kohlenstoffatomen, dic Allyl- oder Benzylgruppe darstellt, und deren Säureadditionssalze mit anorganischen oder organischen Säuren.

4. 2-Amino-6,7,8,9-tetrahydro-5H-thiazolo [4',5':5,4] thieno-[2,3-d] azepin-Derivate der allgemeinen Formel I gemäß Anspruch 1, in der R wie im Anspruch 3 definiert ist, und deren Säurenadditionssalze mit anorganischen oder organischen Säuren.

5. 2-Amino-5,6,7,8-tetrahydro-thiazolo [4',5':5,4] thieno [2,3-c] pyridin-Derivate der allgemeinen Formel I gemäß Anspruch 1, in der R wie im Anspruch 3 definiert ist, und deren Säureadditionssalzc mit anorganischen oder organischen Säuren.

6. 7-Äthyl-2-amino-6,7,8,9-tetrahydro-5H-thiazolo [4',5':5,4]-thieno [2,3-d] azepin und dessen Säureadditionssalze mit anorganischen oder organischen Säuren.

7. physiologisch verträgliche Säureadditionssalze mit anorganischen oder organischen Säuren der Verbindungem gemäß den Ansprüchen 1 bis 6.

8. Arzneimittel, enthaltend eine Verbindung gemäß den Ansprüchen 1 bis 6 oder deren physiologisch verträgliches Säureadditionssalz mit einer anorganischen oder organischen Säure neben einem oder mehreren inerten Trägerstoffen und/oder Verdünnungsmitteln.

9. Verwendung einer Verbindung gemäß den Ansprüchen 1 bis 6 oder deren physiologisch verträgliches Säureadditionssalz zur Herstellung eines Arzneimittels mit einer herzfreque senkenden Wirkung.

10. Verfahren zur Herstellung von Thieno-thiazol-Derivaten der allgemeinen Formel

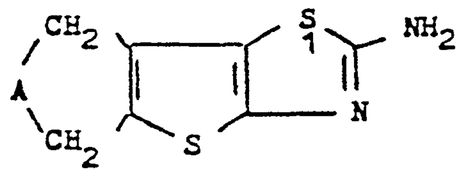

,(I)

gemäß Anspruch 1
und von deren Säureadditionssalzen,
dadurch gekennzeichnet, daß
a) eine gegebenenfalls im Reaktionsgemisch gebildete Verbindung der allgemeinen Formel

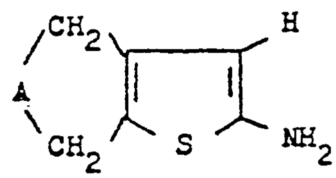

,(II)

in der
A wie eingangs definiert ist, mit gegebenenfalls im Reaktionsgemisch gebildeten Dirhodan umgesetzt wird oder
b) zur Herstellung einer Verbindung der allgemeinen Formel I, in der R eine gegebenenfalls durch eine Alkylgruppe mit 1 bis 4 Kohlenstoffatomen substituierte Methylgruppe darstellt, eine Verbindung der allgemeinen Formel

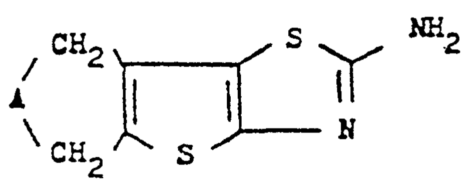

,(III)

in der
A eine Gruppe der Formel

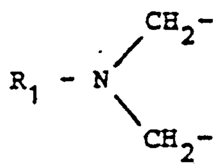 , 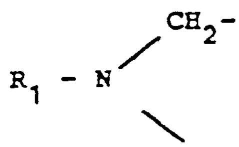 oder 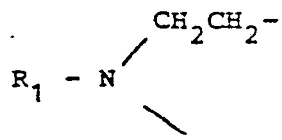

darstellt, wobei
$R_1$ eine Alkanoylgruppe mit 1 bis 5 Kohlenstoffatomen oder einen Kohlensäureesterrest bedeutet, reduziert wird und gewünschtenfalls eine so erhaltene Verbindung der allgemeinen Formel I in ihr Säureadditionssalz, insbesondere in ihr physiologisch verträgliches Säureadditionssalz mit einer anorganischen oder organischen Säure übergeführt wird.

**Patentansprüche** für den Vertrasstaat AT

1. Verfahren zur Herstellung von Thieno-thiazol-Derivaten der allgemeinen Formel

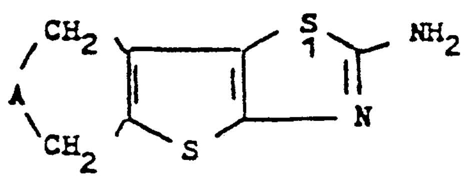 ,(I)

in der
A eine Gruppe der Formel

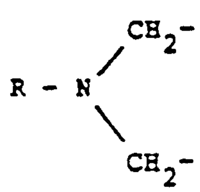 , 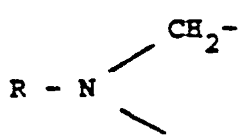 oder 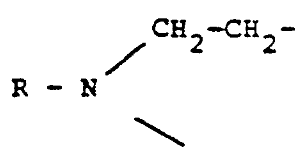

bedeutet, wobei
R ein Wasserstoffatom, eine Alkyl- oder Alkanoylgruppe mit 1 bis 5 Kohlenstoffatomen, die Allyl-, Crotyl-, Penten-2 bzw. 3-ylgruppe, eine Alkoxycarbonylgruppe mit insgesamt 2 bis 5 Kohlenstoffatomen oder eine gegebenenfalls durch ein Halogenatom substituierte Aralkylgruppe mit 7 bis 11 Kohlenstoffatomen darstellt, und von deren Säureadditionssalzen, insbesondere von deren physiologisch verträglichen Säureadditionssalzen mit anorganischen oder organischen Säuren, dadurch gekennzeichnet, daß
a) eine gegebenenfalls im Reaktionsgemisch gebildete Verbindung der allgemeinen Formel

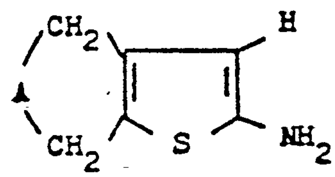

,(II)

in der
A wie eingangs definiert ist, mit gegebenenfalls im Reaktionsgemisch gebildeten Dirhodan umgesetzt wird oder
b) zur Herstellung einer Verbindung der allgemeinen Formel I, in der R eine gegebenenfalls durch eine Alkylgruppe mit 1 bis 4 Kohlenstoffatomen substituiertc Methylgruppe darstellt, eine Verbindung der allgemeinen Formel

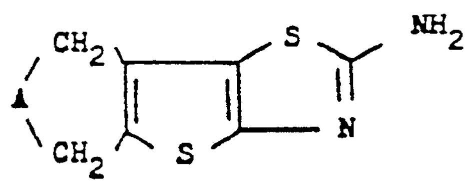

,(III)

in der
A eine Gruppe der Formel

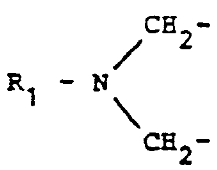

,

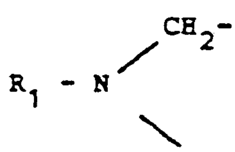

oder

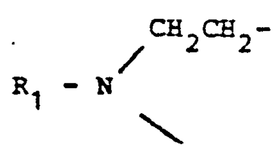

darstellt, wobei
$R_1$ eine Alkanoylgruppe mit 1 bis 5 Kohlenstoffatomen oder einen Kohlensäureesterrest bedeutet, reduziert wird und gewünschtenfalls eine so erhaltene Verbindung der allgemeinen Formel I in ihr Säureadditionssalz, insbesondere in ihr physiologisch verträgliches Säureadditionssalz mit einer anorganischen oder organischen Säure übergeführt wird.

2. Verfahren gemäß Anspruch 1, dsdurch gekennzeichnet, daß die Umsetzung in einem Lösungsmittel durchgeführt wird.

3. Verfahren gemäß den Ansprüchen laund 2, dadurch gekennzeichnet, daß die Umsetzung bei Temperaturen zwischen -5 und 50°C, vorzugsweise jedoch bei Temperaturen zwischen C und 25°C, durchgeführt wird.

4. Verfahren gemäß den Ansprüchen 1b und 2, dadurch gekennzeichnet, daß die Reduktion mit einem Hydrid durchgeführt wird.

5. Verfahren gemäß den Ansprüchen 1b, 2 und 4, dadurch gekennzeichnet, daß die Umsetzung bei Temperaturen zwischen C und 100°C, vorzugsweise jedoch bei der Siedetemperatur des verwendeten Lösungsmittels, durchgeführt wird.

**Claims** for the contracting states BE CH DE FR IT LI LU NL SE

1. Thieno-thiazole derivatives of general formula

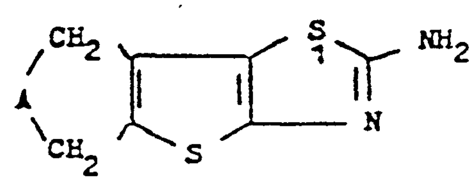

(I)

wherein
A represents a group of formula

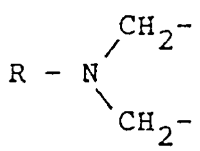

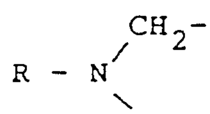

or

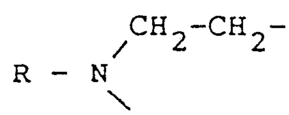

wherein
R represents a hydrogen atom, an alkyl or alkanoyl group with 1 to 5 carbon atoms, the allyl, crotyl, penten-2- or -3-yl group, an alkoxycarbonyl group with a total of 2 to 5 carbon atoms or an aralkyl group with 7 to 11 carbon atoms optionally substituted by a halogen atom, and the acid addition salts thereof.

2. 2-Amino-6,7,8,9-tetrahydro-5H-thiazolo [4',5':5,4]-thieno[2,3-d]azepine, 2-amino-5,6,7,8-tetrahydro-thiazolo[4',5':5,4]thieno[3,2-c]pyridine, 2-amino-5,6,7,8-tetrahydro-thiazolo[4',5':5,4]thieno[2,3-c]pyridine and 2-amino-6,7,8,9-tetrahydro-5H-thiazolo[4',5'.5,4]-thieno[2,3-c]azepine and 2-amino-6,7,8,9-tetrahydro-5H-thiazolo [4',5':5,4] thieno[3,2-c]azepine derivatives of general formula 1 as claimed in claim 1, wherein
R represent a hydrogen atom, an alkyl group with 1 to 4 carbon atoms, an alkanoyl group with 1 to 3 carbon atoms, an alkoxycarbonyl group with a total of 2 to 4 carbon atoms, a benzyl group optionally substituted by a chlorine or bromine atom, or an allyl group, and the acid addition salts thereof.

3. 2-Amino-6,7,8,9-tetrahydro-5H-thiazolo [4',5':5,4]-thieno[2,3-d]azepine and 2-amino-5,6,7,8-tetrahydro-thiazolo[4',5':5,4]thieno[2,3-c]pyridine derivatives of general formula 1 as claimed in claim 1, wherein
R represents a hydrogen atom, an alkyl group with 1 to 3 carbon atoms or an allyl or benzyl group, and the acid addition salts thereof with inorganic or organic acids.

4. 2-Amino-6,7,8,9-tetrahydro-5H-thiazolo[4',5':5,4]-thieno[2,3-d]azepine derivatives of general formula 1 as claimed in claim 1 wherein R is defined as in claim 3, and the acid addition salts thereof with inorganic or organic acids.

5. 2-Amino-5,6,7,8-tetrahydro-thiazolo[4',5':5,4]-thieno[2,3-c]pyridine derivatives of general formula 1 as claimed in claim 1, wherein R is defined as in claim 3, and the acid addition salts thereof with inorganic or organic acids.

6. 7-Ethyl-2-amino-6,7,8,9-tetrahydro-5H-thiazolo-[4',5':5,4]thieno[2,3-d]azepine and the acid addition salts thereof with inorganic or organic acids.

7. Physiologically acceptable acid addition salts with inorganic or organic acids of the compounds as claimed in claims 1 to 6.

8. Pharmaceutical compositions containing a compound as claimed in claims 1 to 6 or a physiologically acceptable acid addition salt thereof with an inorganic or organic acid, together with one or more inert carriers and/or diluents.

9. Use of a compound as claimed in claims 1 to 6 or a physiologically acceptable acid addition salt thereof for preparing a pharmaceutical composition with a heart rate-lowering activity.

10. Process for the preparation of thieno-thiazole derivatives of general formula

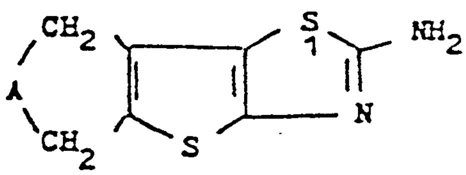

(I)

as claimed in claim 1, and of the acid addition salts thereof, characterised in that
a) a compound of general formula

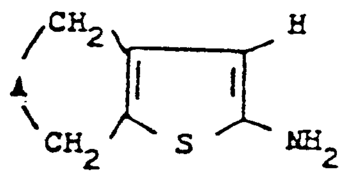

(II)

optionally formed in the reaction mixture, wherein
A is as hereinbefore defined, is reacted with dithiocyanogen optionally formed in the reaction mixture, or
b) in order to prepare a compound of general formula I wherein R represents a methyl group optionally substituted by an alkyl group with 1 to 4 carbon atoms,
a compound of general formula

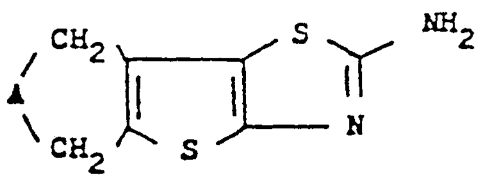

(III)

wherein
A represents a group of formula

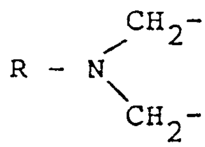 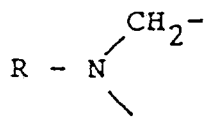 or 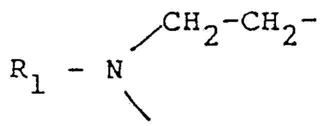

wherein

$R_1$ represents an alkanoyl group with 1 to 5 carbon atoms or a carbonic acid ester group, is reduced, and if desired a compound of general formula 1 thus obtained is converted into an acid addition salt thereof, more particularly a physiologically acceptable acid addition salt thereof with an inorganic or organic acid.

**Claims** for the contracting state:AT

1. Process for the preparation of thieno-thiazole derivatives of general formula

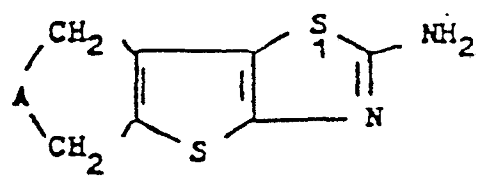

(I)

wherein

A represents a group of formula

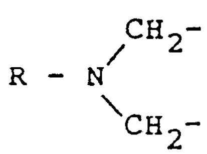 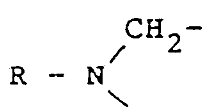 or 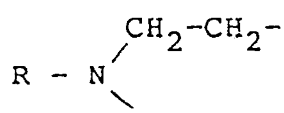

wherein

R represents a hydrogen atom, an alkyl or alkanoyl group with 1 to 5 carbon atoms, the allyl, crotyl, penten-2- or -3-yl group, an alkoxycarbonyl group with a total of 2 to 5 carbon atoms or an aralkyl group with 7 to 11 carbon atoms optionally substituted by a halogen atom, and of the acid addition salts thereof, more particularly the physiologically acceptable acid addition salts thereof with inorganic or organic acids, characterised in that

a) a compound of general formula

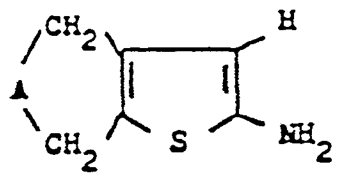

(II)

optionally formed in the reaction mixture, wherein
A is as hereinbefore defined, is reacted with dithiocyanogen optionally formed in the reaction mixture, or

b) in order to prepare a compound of general formula I wherein R represents a methyl group optionally substituted by an alkyl group with 1 to 4 carbon atoms,
a compound of general formula

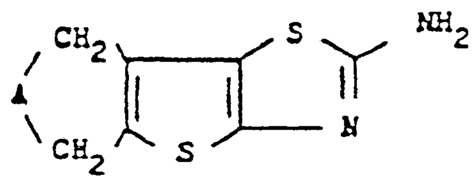

(III)

wherein
A represents a group of formula

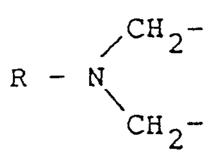

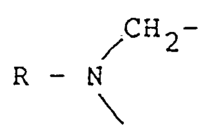

or

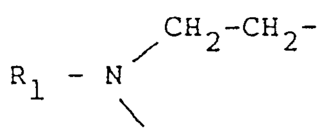

wherein
$R_1$ represents an alkanoyl group with 1 to 5 carbon atoms or a carbonic acid ester group, is reduced, and if desired a compound of general formula 1 thus obtained is converted into an acid addition salt thereof, more particularly a physiologically acceptable acid addition salt thereof with an inorganic or organic acid.

2. Process as claimed in claim 1, characterised in that the reaction is carried out in a solvent.

3. Process as claimed in claims 1a and 2, characterised in that the reaction is carried out at temperatures of between -5 and 50°C, but preferably at temperatures of between 0 and 25°C.

4. Process as claimed in claims 1b and 2, characterised in that the reduction is carried out with a hydride.

5. Process as claimed in claims 1b, 2 and 4, characterised in that the reaction is carried out at temperatures of between 0 and 100°C, but preferably at the boiling temperature of the solvent used.

**Revendications** Pour les Etats contractants: BE, CH, DE, FR, IT, LI, LU, NL, SE.

1. Dérivés du thiéno.-thiazole de formule générale:

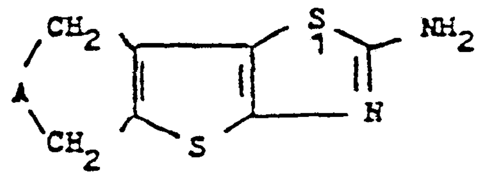

,(I)

dans laquelle:
A représente un groupe de formule:

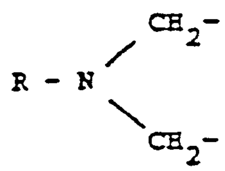

,

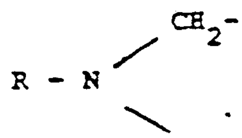

ou

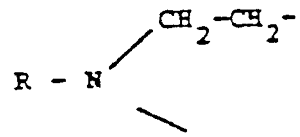

dans lesquelles:

R représente un atome d'hydrogène, un groupe alcoyle ou alcanoyle avec 1 à 5 atomes de carbone, le groupe allyle, crotyle,pentène-2-ou-3-yle, un groupe alcoxycarbonyle avec en tout 2 à 5 atomes de carbone ou un groupe aralcoyle avec 7 à 11 atomes de carbone, éventuellement substitué par un atome d'halogène, et leurs sels d'addition d'acides.

2. Dérivés de la 2-amino-6,7,8,9-tétrahydro-5H-thiazolo[4',5':5,4]thiéno-[2,3-d]azépine, de la 2-amino-5,6,7,8-tétrahydro-thiazolo-[4',5':5,4]thiéno[3,2-c]pyri-dine, de la 2-amino-5,6,7,8-tétrahydro-thiazolo[4',5':5,4]-thiéno-[2,3-c]pyridine et de la 2-amino-6,7,8,9-tétrahydro-5H-thiazolo[4',5':5,4]thiéno[2,3-c]azépine et de la 2-amino-6,7,8,9-tétrahydro-5H-thiazolo[4' 5':5,4]thiéno- [3,2-c] aze-pine, de formule générale I selon la revendication 1, dans laquelle:

R représente un atome d'hydrogène, un groupe alcoyle avec 1 à 4 atomes de carbone, un groupe alcanoyle avec 1 à 3 atomes de carbone, un groupe alcoxycarbonyle avec en tout 2 à 4 atomes de carbone, un groupe benzyle éventuellement substitué par un atome de chlore ou de brome ou un groupe allyle, et leurs sels d'addition d'acides.

3. Dérivés de la 2-amino-6,7,8,9-tétrahydro-5H-thiazolo[4',5':5,4]thiéno-[2,3-d]azépine et de la 2-amino-5,6,7,8-tétrahydro-thiazolo[4',5':5,4]thiéno[2,3-c]pyridine de formule générale I selon la revendication 1, dans laquelle:

R représente un atome d'hydrogène, un groupe alcoyle avec 1 à 3 atomes de carbone, le groupe allyle ou benzyle, et leurs sels d'addition d'acides avec des acides minéraux ou organiques.

4. Dérivés de la 2-amino-6,7,8,9-tétrahydro-5H-thiazolo[4',5':5,4]thiéno-[2,3-d]azépine de formule générale I selon la revendication 1, dans laquelle R est défini comme dans la revendication 3, et leurs sels d'addition d'acides avec des acides minéraux ou organiques.

5. Dérivés de la 2-amino-5,6,7,8-tétrahydro-thia-zolo[4',5':5,4]thiéno [2,3-c] pyridine de formule générale I selon la revendication 1, dans laquelle R est défini comme dans la revendication 3, et leurs sels d'addition d'acides avec des acides minéraux ou organiques.

6. La 7-éthyl-2-amino-6,7,8,9-tétrahydro-5H-thia-zolo[4',5':5,4]thiéno [2,3-d] azépine et ses sels d'addition d'acides avec des acides minéraux ou organiques.

7. Sels d'addition d'acides physiologiquement supportables avec des acides minéraux ou organiques des composés selon les revendications 1 à 6.

8. Médicament contenant un composé selon les revendications 1 à 6 ou son sel d'addition d'acide physiologiquement supportable avec un acide minéral ou organique conjointement à un ou plusieurs excipients et/ou diluants inertes.

9. Utilisation d'un composé selon les revendications 1 à 6 ou de son sel d'addition d'acide physiologiquement supportable pour la préparation d'un médicament présentant une activité d'abaissement de la fréquence cardiaque.

10. Procédé pour la préparation de dérivés du thiéno-thiazole de formule générale:

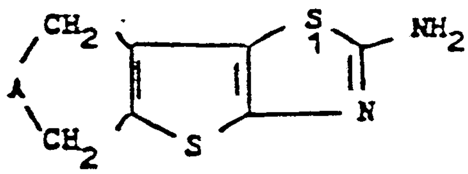

,(I)

selon la revendication 1, caractérisé en ce que:
a) on fait réagir un composé, éventuellement formé dans le mélange réactionnel, de formule générale:

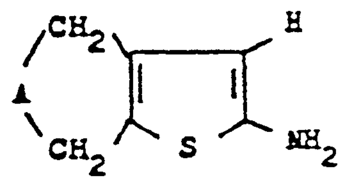

,(II)

dans laquelle:
A est défini comme au début, avec du dirhodane éventuellement formé dans le mélange réactionnel ou
b) pour la préparation d'un composé de formule générale I dans laquelle R représente un groupe méthyle éventuellement substitué par un groupe alcoyle avec 1 à 4 atomes de carbone, on réduit un composé de formule générale:

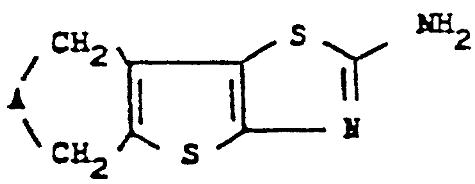

,(III)

dans laquelle:
A représente un groupe de formule:

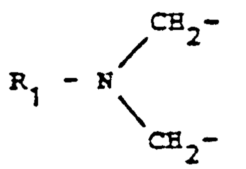 , 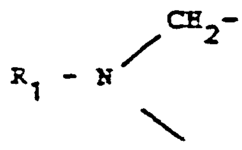 ou 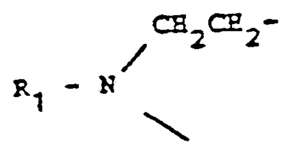

dans lesquelles:

R représente un groupe alcanoyle avec 1 à 5 atomes de carbone ou un radical d'ester carbonique et si on le désire, on transforme un composé ainsi obtenu de formule générale I en son sel d'addition d'acide, en particulier en son sel d'addition d'acide physiologiquement supportable avec un acide minéral ou organique.

**Revendications** Pour l'Etat contractant: AT

1. Procédé pour la préparation de dérivés du thiéno-thiazole de formule générale:

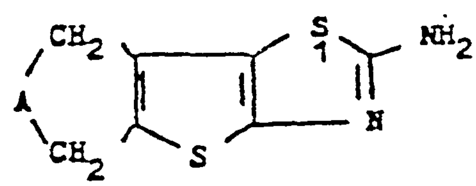 ,(I)

dans laquelle:

A représente un groupe de formule:

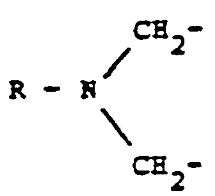 , 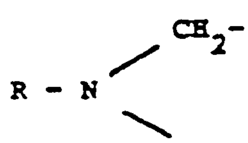 ou 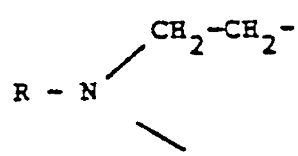

dans lesquelles:

R représente un atome d'hydrogène, un groupe alcoyle ou alcanoyle avec 1 à 5 atomes de carbone, le groupe allyle, crotyle, pentène-2-ou -3-yle, un groupe alcoxycarbonyle avec en tout 2 à 5 atomes de carbone ou un groupe aralcoyle avec 7 à 11 atomes de carbone, éventuellement substitue par un atome d'halogène,et de leurs sels d'addition d'acides, en particulier de leurs sels d'addition d'acides physiologiquement supportables avec des acides minéraux ou organiques, caractérisé en ce que:

a) on fait réagir un composé, éventuellement formé dans le mélange réactionnel, de formule générale:

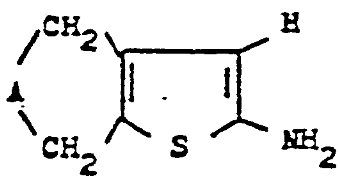

,(II)

dans laquelle:

A est défini comme au début, avec du dirhodane éventuellement formé dans le mélange réactionnel ou

b) pour la préparation d'un composé de formule générale I dans laquelle R représente un groupe méthyle éventuellement substitué par un groupe alcoyle avec 1 à 4 atomes de carbone, on réduit un composé de formule générale:

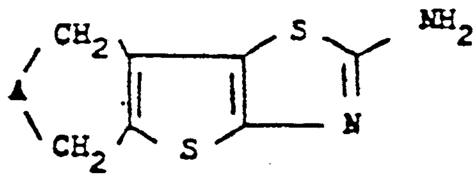

,(III)

dans laquelle:

A représente un groupe de formule:

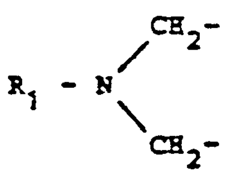

,

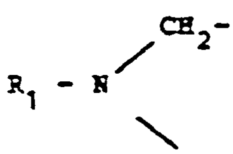

ou

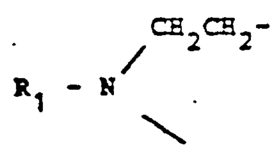

dans lesquelles:

R représente un groupe alcanoyle avec 1 à 5 atomes de carbone ou un radical d'ester d'acide carbonique, et si on le désire, on transforme un composé ainsi obtenu de formule générale I en son sel d'addition d'acide, en particulier en son sel d'addition d'acide physiologiquement supportable avec un acide minéral ou organique.

2. Procédé selon la revendication 1, caractérisé en ce que la réaction est effectuée dans un solvant.

3. Procédé selon les revendications 1a et 2, caractérisé en ce que la réaction est effectuée à des températures entre -5 et 50°C, de préférence cependant à des températures entre 0 et 25°C.

4. Procédé selon les revendications 1b et 2, caractérisé en ce que la réduction est effectuée au moyen d'un hydrure.

5. Procédé selon les revendications 1b, 2. et 4, caractérisé en ce que la réaction est effectuée à des temperatures entre 0 et 100°C, de préférence cependant à la température d'ébullition du solvant utilisé.